# EUROPÄISCHE PATENTSCHRIFT

(11) **EP 3 308 734 B1**
(45) Veröffentlichungstag und Bekanntmachung des Hinweises auf die Patenterteilung: **27.04.2022**
(21) Anmeldenummer: 17195980.2
(22) Anmeldetag: 11.10.2017
(51) Int. Cl.: A61B 18/22, A61B 17/3211, A61B 18/20

(54) **OPTISCHES SKALPELL UND CHIRURGISCHE SCHNEIDEVORRICHTUNG**
OPTICAL SCALPEL AND SURGICAL CUTTING DEVICE
SCALPEL OPTIQUE ET DISPOSITIF DE COUPE CHIRURGICAL

(30) Priorität: 11.10.2016 DE 102016119360
(43) Veröffentlichungstag der Anmeldung: 18.04.2018
(73) Patentinhaber: biolitec Unternehmensbeteiligungs II AG, 1030 Wien (AT)
(72) Erfinder: KLAMANN, Karl, 90542 Eckental (DE)
(74) Vertreter: Herrmann, Franz

(56) Entgegenhaltungen:
- EP-A1- 3 067 005
- EP-B1- 3 067 005
- WO-A1-01/87176
- DE-A1- 19 803 720
- DE-A1-102011 122 209
- DE-B3-102004 007 120
- US-A1- 2014 180 264
- US-B1- 6 673 065

## Beschreibung

Die vorliegende Erfindung betrifft ein optisches Skalpell bestehend aus zumindest einem Lichtleiter, mit einem proximalen und einem, vom proximalen Ende abgewendeten, distalen Ende, wobei an dessen proximalen Ende zumindest eine Bearbeitungslichtquelle anschließbar ist.

Die Erfindung betrifft ferner eine chirurgische Schneidevorrichtung.

Die Verwendung von hochenergetischer Strahlung, insbesondere von Laserstrahlung, im medizinischen Bereich, ist sowohl für die Behandlung von außen (ab externo), als auch bei endoskopischen Behandlungen (ab interno) entsprechend bekannt. Als Vorteil ist dabei anzuführen, dass die Verwendung von energiereichem Licht, insbesondere Laserstrahlung, die Applikation von photothermischen, bzw. photoinduzierten Eigenschaften erlaubt. Der teilweise gewünschte Gewebetrennungseffekt lässt sich so alleinstehend ebenso erzielen, wie eine Kombination mit photoinduzierten chemischen, physikalischen oder biologischen Prozessen. Im Vergleich zur Verwendung einer klassischen mechanisch agierenden Klinge zur Gewebetrennung, lassen sich weitere Eigenschaften einzeln oder kombiniert hinzufügen. Ein weiterer großer Vorteil bei der Verwendung von Laserstrahlung im chirurgischen Sinne ist, dass Blutgefäße beim Schnitt direkt verschlossen werden können und es somit zu nahezu keinen Blutungen kommt. Außerdem kann der Wundrand gleichzeitig erhitzt und so desinfiziert werden.

Um die Laserstrahlung, welche beispielsweise von einem Festkörperlaser emittiert wird, entsprechend nutzen zu können, werden sogenannte Laser-Skalpelle genutzt.

Beispielsweise zeigt die EP 0 372 362 B1 ein Laserskalpell zur chirurgischen Anwendung, welches aufgrund der Werkstoffwahl eine möglichst hohe Arbeitstemperatur ermöglicht und eine gewisse Unempfindlichkeit auf Thermoschocks aufweist. Das bekannte Laserskalpell zeigt einen Aufbau, bei welchem die Skalpellspitze herausnehmbar, also vom Lichtleiter abtrennbar, ausgeführt ist. Dies ermöglicht eine entsprechend einfache und gründliche Reinigung. Um einen möglichst effektiven Übergang der Laserstrahlung vom Lichtleiter zur Skalpellspitze zu gewährleisten, ist die Skalpellspitze mit einer Bohrung versehen, in welche der Lichtleiter einführbar ist. Nachteilig ist der relativ komplizierte Aufbau. Weiters kommt es zwangsweise, durch die Einkopplung in die Skalpellspitze, zu entsprechenden Leistungsverlusten. Ein derartiger Aufbau kann des Weiteren zu einem erhöhten Aufwand bei der Reinigung führen.

Die DE 10 2009 011 587 A1 zeigt einen Skalpellkörper, welcher aufgrund seiner Geometrie einen hohen Anteil der eingekoppelten Laserstrahlung an der Spitze des Skalpellkörper zur Verfügung stellen kann. Dabei liegt das Hauptaugenmerk auf einem definierten Einkoppelwinkel, über welchen die Laserstrahlung in den Skalpellkörper eingekoppelt wird. Der Lichtleiter, über welchen die Laserstrahlung geleitet wird, und der Skalpellkörper sind dabei getrennt ausgeführt. Dies hat den Nachteil, dass eine entsprechend genaue Positionierung der beiden zueinander notwendig ist, um die geometrischen Vorteile entsprechend nutzen zu können. Auch hier kommt es durch die Einkopplung zu den bereits erwähnten Leistungsverlusten.

Aus der EP 3 067 005 A1 ist ferner eine Vorrichtung für die Laserchirurgie bekannt, bei der ein Lichtwellenleiter eine Laserquelle mit verschiedenen Handstücken verbindet, die jeweils mit einer optische Faser versehen sind. Die in den Handstücken vorgesehenen optischen Faser weisen distale Enden auf, die herkömmlichen mechanischen Skalpells nachgebildet sind.

Ausgehend von diesem Stand der Technik liegt der Erfindung die Aufgabe zugrunde, ein chirurgisches Instrument auf Basis eines Lichtleiters auszubilden, welches ähnlich einem Skalpell wirkt, das wahlweise auch als Kauter genutzt werden kann, möglichst einfach im Aufbau und kostengünstig herzustellen ist.

Die Aufgabe wird gemäß der vorliegenden Erfindung durch die Merkmale des Anspruchs gelöst.

Dadurch, dass das distale Ende des Lichtleiters als Skalpellendung ausgebildet ist, sind das eigentliche Skalpell und der Lichtleiter einstückig ausgebildet sind. Durch die Einstückigkeit ergibt sich ein denkbar einfacher Aufbau.

Das als Skalpellendung ausgebildet distale Ende des Lichtleiters kann zumindest eine am distalen Ende des Lichtleiters angeordnete Ecke aufweisen, in der sich zumindest drei Kanten schneiden, von denen zumindest eine als Schneidekante ausgebildet ist, die sich von der Ecke aus wenigstens abschnittsweise in Richtung des proximalen Endes des Lichtleiters erstreckt. Dadurch ist sichergestellt, dass die Schneidekante über die gesamte Skalpellendung hinweg ausreichend abgestützt ist.

Bei einer Ausführungsform ist vorgesehen dass im Querschnitt des Lichtleiters die Ecke der Skalpellendung weiter von der Mitte des Lichtleiters entfernt ist als von der Außenseite des Lichtleiters. Dadurch wird sichergestellt, dass die Schneidekante in die der Schneiderichtung entgegengesetzte Richtung mit so viel Material unterlegt ist, dass eine ausreichende Festigkeit der Schneidkante gewährleistet ist.

Bei einer weiteren Ausführungsform ist vorgesehen, dass sich die Ecke der Skalpellendung im Querschnitt auf der Außenseite des Lichtleiters befindet. Die Ecke der Skalpellendung kann sich insbesondere auf der Umfangsfläche der zylinderförmigen Grundform des Lichtleiters befinden. Dadurch weist die Schneidekante die maximal mögliche Festigkeit auf.

Bei einer weiteren Ausführungsform ist vorgesehen, dass sich eine vom distalen Ende des Lichtleiters ausgehende Schnittfase im Querschnitt des Lichtleiters zu der Schneidekante hin verjüngt. Dadurch ergibt sich eine langgestreckte, seitliche Schnittkante, mit der auch Schnitte mit großer Schnitttiefe möglich sind.

Ferner kann vorgesehen sein, dass die Skalpellendung durch eine keilförmige Verjüngung des Lichtleiters ausgebildet ist. Durch zumindest eine dabei gebildete Ecke ist eine am distalen Ende des Lichtleiters liegende Schnittfase gebildet. Dadurch kann bereits durch ein zweiseitiges Bearbeiten des Lichtleiters eine entsprechend vorteilhaft zu nutzende Skalpellendung gebildet werden. Insofern ist diese Ausführungsform besonders einfach herstellbar.

Am distalen Ende des Lichtleiters ist eine quer zur Längsachse des Lichtleiters ausgerichtete Stirnfläche ausgebildet . Dadurch tritt das Licht aus der Lichtfaser überwiegend über die Stirnfläche aus und kann dort die nötige Koagulation der Schnittflächen bewirken. Die Stirnfläche kann insbesondere ungefähr im rechten Winkel zur Längsachse ausgerichtet sein und/oder eine Fläche zwischen der Hälfte und einem Drittel, Viertel, Fünftel, Sechstel oder Achtel der Querschnittsfläche des Lichtleiters einnehmen.

Weiter kann vorgesehen sein, dass die am distalen Ende des Lichtleiters normal zu dessen Längserstreckung liegende Stirnfläche der Skalpellendung als Polygon ausgeführt ist. Durch zumindest eine Ecke der Stirnfläche, ist eine am distalen Ende des Lichtleiters liegende, keilförmige Schnittfase gebildet. Bei dieser Ausführungsform ist zum einen die Schnittfase besonders lang, so dass auch tiefe Schnitte vorgenommen werden können.

Weiter kann vorgesehen sein, dass der zumindest eine Lichtleiter über seine gesamte Länge einen gleichbleibenden polygonen Querschnitt aufweist. Durch zumindest eine Ecke des polygonen Querschnittes ist eine keilförmige Schnittfase gebildet, welche zumindest annähernd in Richtung der Längserstreckung des Lichtleiters verläuft. Auf diese Weise kann eine bereits für die Lichtleiter vorgewählte Form vorteilhaft genutzt werden. Ein zusätzliches Bearbeiten jenes Bereichs, durch welchen die Skalpellendung und deren polygone Stirnfläche gebildet ist, ist nicht notwendig.

Eine weitere Ausführungsform sieht vor, dass der polygone Querschnitt der Skalpellendung derart ausgestaltet ist, dass zumindest zwei Schnittfasen mit zumindest annähernd gleichem oder unterschiedlichem Keilwinkel gebildet sind. Bei zwei gleichartigen Keilwinkeln kann sich beispielsweise der Vorteil der bidirektionalen Nutzung ergeben.

Unterschiedliche Keilwinkel können beispielsweise für unterschiedliche Verfahren angewandt werden. Dabei ist vorteilhaft vorgesehen, dass der Keilwinkel einer Schnittfase zwischen 5° und 120°, insbesondere zwischen 25° und 50° gewählt ist. Zum einen lässt dieser Winkelbereich eine vorteilhafte Fokussierung der Lichtenergie zu. Zum anderen wird bei entsprechender Winkelwahl die mechanische Schneidwirkung unterstützt.

Das optische Skalpell kann Teil einer chirurgischen Schneidevorrichtung sein, in der das optische Skalpell in an die Bearbeitungslichtquelle angeschlossen ist.

Bei einer Ausführungsform ist die eine Bearbeitungslichtquelle von einem Laser gebildet. Dadurch können Eigenschaften des Laserlichts, wie hohe Energiedichte und geringe Divergenz vorteilhaft genutzt werden.

Weiters kann eine weitere Markierungslichtquelle, zur Markierung eines Behandlungsbereiches vorgesehen sein. Diese erlaubt ein erleichtertes Positionieren der Skalpellendung relativ zum zu behandelnden Gewebe.

Das von der Markierlichtquelle ausgehende Markierlicht mit Hilfe einer Koppelvorrichtung zusammen mit der Laserstrahlung dem Lichtleiter zugeführt sein. Dadurch tritt das Markierlicht im Wesentlichen an der Stelle aus dem Lichtleiter aus, an der auch das Bearbeitungslicht den Lichtleiter verlässt. Insofern wird der Bereich auf den die Laserstrahlung einwirkt für den Benutzer zuverlässig markiert.

Die gegenständliche Erfindung wird nachfolgend unter Bezugnahme auf die Figu-ren 1 bis 10 erläutert, die beispielhaft, schematisch und nicht einschränkend verschiedene Ausgestaltungen der Erfindung zeigen. Dabei zeigt
- Figur 1: eine chirurgische Schneidevorrichtung,
- Figur 2: ein Ausführungsbeispiel der Skalpellendung eines optischen Skalpells,
- Figur 3: ein abgewandeltes Ausführungsbeispiel der Skalpellendung,
- Figur 4: ein Längsschnitt durch das in die in Figur 3 dargestellte Ausführungsbeispiel einer Skalpellendung,
- Figur 5: eine Darstellung, die die Wirkweise einer Skalpellendung veranschaulicht,
- Figur 6: eine Vorderansicht auf die Skalpellendung aus Figur 5,
- Figur 7: eine Seitenansicht eines weiteren Ausführungsbeispiels des optischen Skalpells,
- Figuren 8 bis 10: Vorderansichten von verschiedenen beispielhaften Skalpellendungen aus Richtung A in Fig. 7

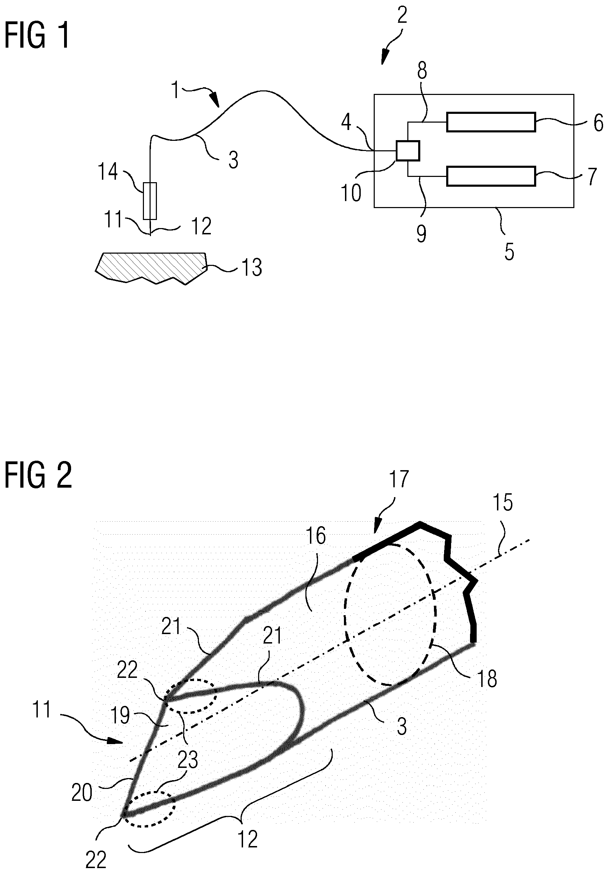

Figur 1 zeigt ein optisches Skalpell 1 einer chirurgischen Scheidevorrichtung 2. Das optische Skalpell umfasst einen Lichtleiter 3, der mit seinem proximalen Ende 4 an Versorgungseinheit 5 angeschlossen ist. Der Lichtleiter 3 ist üblicherweise eine Glasfaser oder eine teilweise oder ganz aus Kunststoff hergestellte Faser. In der Versorgungseinheit 5 befindet sich eine Bearbeitungslichtquelle 6 und eine Markierungslichtquelle 7. Eine von der Bearbeitungslichtquelle 6 abgegebene Laserstrahlung 8 und ein von der Markierlichtquelle 7 abgegebenes Markierlicht 9 werden über eine geeignete Koppelvorrichtung 10 zusammen dem Lichtleiter 3 zugeführt werden. Die Laserstrahlung 8 und das Markierlicht 9 wird vom Lichtleiter 3 zu einem distalen Ende 11 geführt, das als Skalpellendung 12 ausgebildet ist. Die an der Skalpellendung 12 austretende Laserstrahlung 8 wirkt auf zu behandelndes Gewebe 13 ein. Das Markierlicht 9 dient dazu den zu behandelnden Bereich vor dem Zuführen der Laserstrahlung 8 zu markieren. Das Markierlicht 9 kann daher alternierend mit der Laserstrahlung 8 zugeführt werden oder dauerhaft, auch während der Anwendung der Laserstrahlung 8, zugeführt werden. Zum Führen des optischen Skalpells 1 kann eine Haltevorrichtung 14 vorgesehen sein.

Bei dem in Figur 1 dargestellten Ausführungsbeispiel des optischen Skalpells 1 werden Laserstrahlung 8 und Markierlicht 9 über denselben Lichtleiter 3 geführt. Es ist jedoch auch denkbar für das Markierlicht 9 einen eigenen Lichtleiter 3 vorzusehen, mit dem das Markierlicht 9 dem Behandlungsort zugeführt wird Die Bearbeitungslichtquelle 6 umfasst in der Regel einen Laserstrahlung 8 emittierenden Laser. Anstelle eines derartigen Lasers kann aber auch eine inkohärentes Licht abgebende Lichtquelle verwendet werden. Insofern ist die Nutzung der Laserstrahlung 8 nicht zwingend notwendig. Dennoch wird in weiterer Folge in diesem Zusammenhang, lediglich von Laserstrahlung 8 gesprochen. Dies ist jedoch keinesfalls einschränkend zu sehen. Zur Wirkweise der Laserstrahlung 8 sei angemerkt, dass bekanntermaßen bei höherer Intensität der Laserstrahlung 8 die Wechselwirkung mit dem zu behandelnden Gewebe 13 zunimmt. Die durch Laserstrahlung 8 eingestrahlte Lichtenergie wird zum einen in Wärme umgewandelt, sodass es, ab einer gewissen Temperatur, zu einer strukturellen Veränderung der Proteine im Gewebe 13 kommt (Denaturierung). Dadurch tritt insbesondere ein erwünschter, blutstillender Effekt ein.

Durch das Einwirken der Laserstrahlung 8 des Bearbeitungslasers 6, kommt es weiter zum Effekt, dass das im Gewebe 13 enthaltene Wasser verdampft. Diese Verdampfung kann zum Teil explosionsartig verlaufen. Das zurückbleibende Gewebe 13 besitzt ein entsprechend kleineres Volumen. Daher kann der beschriebene Effekt zum Trennen von Gewebeschichten benutzt werden, wobei es gleichzeitig zum bereits erwähnten Gerinnungseffekt (Kauterisation) im Wundbereich kommen kann. Natürlich sind, abhängig von der Wellenlänge der Laserstrahlung 8, der eingebrachten Energie oder Intensität und dem bestrahlten Applikationsfeld auch andere bekannte Effekte, wie beispielsweise weitere photoablative, -disruptive, -dynamische,-biologische oder ähnliche Wechselwirkungen denkbar.

Bezüglich der soeben erwähnten Wellenlänge der Laserstrahlung 8 sei angemerkt, das prinzipiell die Nutzung in einem Bereich zwischen 30 nm und 50.000 nm möglich ist, wobei vorzugsweise ein Bereich zwischen 150 nm und 10.600 nm und besonders vorteilhaft ein Bereich zwischen 400 nm und 2000 nm zur Anwendung kommt. Insbesondere im zuletzt genannten Bereich kommt ein hoher Absorptionsgrad des im Blutplasma enthaltenen Wassers positiv zum Tragen. Der denkbare Leistungsbereich liegt zwischen 0,1 W bis 100 GW im sogenannten "peak power pulsed mode". Vorzugsweise kommen Leistungen zwischen 0,1W und 10 MW zur Anwendung.

Aufgrund der Skalpellendung 12 kann auch ein mechanisches Schneiden des Gewebes 13 bewirkt werden. Die Skalpellendung 12 ist insbesondere derart geformt, das ein mechanisches Trennen des Gewebes 13 mit und auch ohne die Laserstrahlung 8 ermöglicht wird.

Die zusätzliche Nutzung der Laserstrahlung 8 führt neben dem mechanischen Einwirken der Skalpellendung 12 zu einer zusätzlichen Interaktion mit dem Gewebe 13 wie beispielsweise der Verschluss, verletzter Gefäße. Dabei dient die genutzte Laserstrahlung 8 jedoch nicht lediglich dazu die Skalpellendung 12 zu erwärmen. Vielmehr wird die Laserstrahlung 8 wird an der Skalpellendung 12 emittiert und es kommt zur bereits eingangs erwähnten Wechselwirkung mit dem Gewebe 13.

Besonders hervorzuheben ist, dass der Lichtleiter 3 und die Skalpellendung 12 einstückig ausgebildet sind. Unter Einstückigkeit ist hier zu verstehen, dass der Lichtleiter 3 und die Skalpellendung 12 aus ein und demselben Werkstück oder Bauteil gebildet sind. Beispielsweise ist denkbar, dass die Skalpellendung 12 durch entsprechendes Bearbeiten oder Umformen des verwendeten Lichtleiters 3 im Bereich der Skalpellendung 12 gebildet wird. Dabei kommen selbstverständlich unterschiedlichste, entsprechend bekannte Bearbeitungs- und Umformtechniken in Betracht, die dem Fachmann grundsätzlich bekannt sind.

Durch die Einstückigkeit ergibt sich ein denkbar einfacher Aufbau. Reinigung, Desinfektion und dergleichen, sind aufgrund der Einstückigkeit wesentlich vereinfacht. Durch die Einsparung diverser Verbindungselemente ergibt sich natürlich auch ein entsprechender Kostenvorteil. Selbstverständlich kann in bekannter Weise am besagten proximalen Ende 4 des Lichtleiters 3 ein in Figur 1 nicht dargestelltes Verbindungselement vorgesehen sein, welches ermöglicht, den Lichtleiter 3 mit dem Gehäuse 15 zu verbinden.

Die erwähnte, zumindest eine Bearbeitungslichtquelle 6 wird in der Regel durch wenigstens einen Laser gebildet, wobei natürlich unterschiedlichste Laserquellen und Wellenlängen mit unterschiedlichen Leistungen, je nach ihren gewünschten medizinischen Eigenschaften, genutzt werden können.

Lediglich beispielhaft seien häufig genutzte Laserquellen wie NdYAG, ErbYAG Laser, GaAs Diodenlaser, oder auch CO₂ Laser genannt, welche je nach erwünschter Anwendung und damit gewählten Wellenlängen- und Leistungsbereich zur Koagulation, Sublimation oder Erwärmung des zu behandelnden Gewebes 13 gewählt werden können.

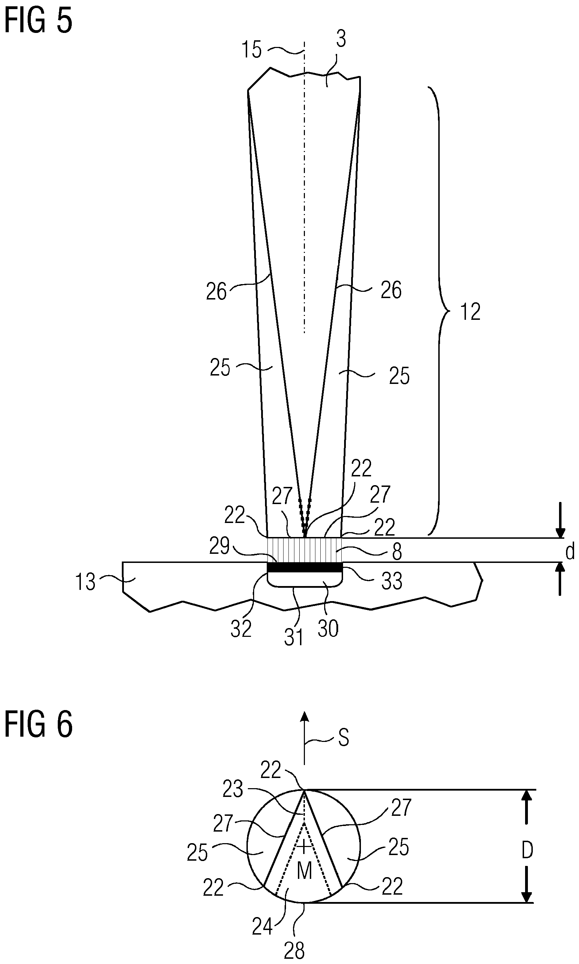

Neben der eben erwähnten Bearbeitungslichtquelle 6 ist vorgesehen, dass zumindest eine weitere Markierungslichtquelle 7 zur Markierung eines Behandlungsbereiches vorgesehen ist. Natürlich kann es sich bei dieser Markierungslichtquelle 7 ebenfalls um einen Laser handeln, wobei der gewählte Wellenlängenbereich und Leistungsbereich ein optisches Markieren auf dem zu behandelnden Gewebe 13 ermöglicht. Eine Wechselwirkung mit dem Gewebe 13, wie es für den Laser der Bearbeitungslichtquelle 6 beschrieben wurde ist dabei nicht vorgesehen. Mithilfe der Markierungslichtquelle 7 wird somit ein Positionieren der Skalpellendung 12, relativ zu einer Oberfläche des zu behandelnden Gewebes 13 erleichtert. Vorzugsweise ist die Markierungslichtquelle 7 in ihren Eigenschaften, insbesondere bezüglich der Strahldivergenz des von ihr abgegebenen Markierlichtes 9, derart gewählt, das eine gebildete Markierung die gleiche Geometrie aufweist wie ein durch die Laserstrahlung 8 gebildeter, in Figur 5 dargestellter Brennfleck 29 auf der Oberfläche des Gewebes 13.

Auf diese Weise ist ein Wirkbereich der Laserstrahlung 8 anhand der beschriebenen Markierung abschätzbar.

Das Markierlicht 9 der Markierungslichtquelle 7 kann während der Abgabe der Laserstrahlung 8 weiter an die Oberfläche des Gewebes 13 abgegeben werden, oder die Abgabe des Markierlicht 9 kann währenddessen auch unterbrochen werden, insbesondere wenn die Laserstrahlung 8 Licht im sichtbaren Wellenlängenbereich umfasst. Falls dagegen die Laserstrahlung 8 in einem nicht sichtbaren Wellenlängenbereich liegt, kann das Markierlicht 9 beispielsweise auch zur Kennzeichnung der Emission der Laserstrahlung 8 verwendet werden und immer dann aus der Skalpellendung 12 austreten, wenn auch Laserstrahlung 8 aus der Skalpellendung 12 austritt.

Figur 2 zeigt eine perspektivische Ansicht der Skalpellendung 12 des optischen Skalpells 1. Die Skalpellendung 12 wird dabei durch eine keilförmige Verjüngung des Lichtleiters 3 ausgebildet. Hinsichtlich des Lichtleiters 3 ist in Figur 2 der derzeit übliche industrielle Standard dargestellt. Der Lichtleiter 3 erstreckt sich entlang einer Längsachse 15. Eine Außenseite 16 des Lichtleiters 3 weist dabei entlang einer Längsachse 15 eine zylindrische Grundform 17 auf. Die Grundform 17 verfügt insbesondere einen zumindest annähernd kreisförmigen Querschnitt 18. Durch ein, wie in Figur 2 lediglich beispielhaft dargestellt, zweiseitiges Bearbeiten des Lichtleiters 3 kann bereits eine entsprechend vorteilhaft zu nutzende Skalpellendung 2 gebildet werden. Durch die Bearbeitung des Lichtleiters 3 werden zwei Keilflächen 19 gebildet, die sich in einer spitzen Vorderkante 20 schneiden. In seitliche Richtung sind die Keilflächen 19 jeweils durch eine Seitenkante 21 begrenzt, die jeweils zumindest näherungsweise parabelförmig verläuft und die jeweilige Keilfläche 19 von der Außenseite 16 abgrenzt. Die Vorderkante 20 erstreckt sich über den gesamten Querschnitt 18 des Lichtleiters 3. An den beiden äußeren Enden der Vorderkante 20 bildet die Vorderkante 20 mit den Seitenkanten 21 Ecken 22. Die Ecken 22 liegen somit auf der Außenseite 16 des Lichtleiters 3. An zumindest einer der Ecken 22 ist eine am distalen Ende 11 des Lichtleiters 3 liegende Schnittfase 23 gebildet, beispielsweise dort wo die Seitenkanten 21 mit den gestrichelt eingezeichneten Kreisen gekennzeichnet sind. Dort üben die Seitenkanten 21 die Funktion einer Schneidekante aus.

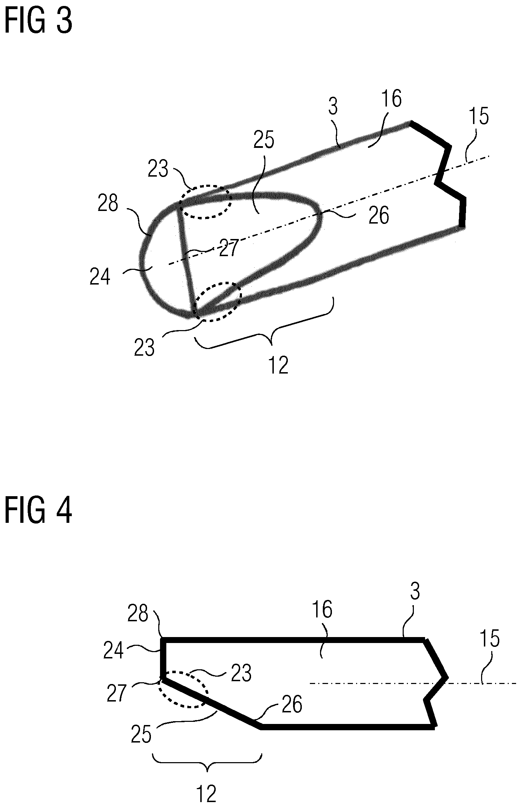

In den Figuren 3 und 4 ist ein weiteres Ausführungsbeispiel dargestellt. Bei diesem Ausführungsbeispiel ist das distale Ende 11 des Lichtleiters 3 nur von einer Seite bearbeitet worden. Weist, wie beispielhaft in den Figuren 3 und 4 dargestellt, der ursprüngliche Lichtleiter 3 die zylindrische Grundform 17 auf, so wird dessen distales Ende 11 von der Außenseite 16 und einer kreisförmigen Stirnfläche 24 begrenzt. Durch die zuvor erwähnte einseitige Bearbeitung des Lichtleiters 3 wird an dessen distalem Ende 11 eine Abschrägungsfläche 25 gebildet. Die Abschrägungsfläche 25 ist zur Außenseite 16 hin durch Seitenkanten 26 und zur Stirnfläche 24 hin durch die Vorderkante 27 begrenzt. Infolgedessen weist, wie insbesondere in Figur 3 erkennbar, die Stirnfläche 24 keinen kreisförmigen Umriss mehr auf, sondern hat die Gestalt eines Kreissegments, das zur Außenseite 16 hin durch eine Außenkante 28 begrenzt ist. Im Schnittpunkt von Seitenkante 26, Vorderkante 27 und Außenkante 28 sind die Ecken 22 gebildet. Dabei wird am distalen Ende 11 des Lichtleiters 3 jeweils eine Ecke 22 an jenem Punkt gebildet, in welchem die Stirnfläche 24 des Lichtleiters 3, die Außenseite 16 des Lichtleiters 3 und die erwähnte Abschrägungsfläche 25 aufeinandertreffen. Auf diese Weise wird am distalen Ende 11 des Lichtleiters 3 eine erfindungsgemäße Skalpellendung 12 ausgebildet. Selbstverständlich sind dabei auch mehrere Bearbeitungsflächen 21, wie beispielhaft in Figur 2 gezeigt, möglich.

Figur 5 veranschaulicht die Wirkungsweise des optischen Skalpells 1 anhand eines Ausführungsbeispiels mit zwei Abschrägungsflächen 25, deren auf den Querschnitt 18 projizierten Normalen einen Winkel kleiner 180° einnehmen. Die in Figur 5 dargestellte Skalpellendung 12 wurde beispielsweise durch eine zweifache Bearbeitung des distalen Endes 11 eines ursprünglich zylindrischen Lichtleiters 3 gebildet. Infolge ergeben sich zwei Abschrägungsflächen 25 mit jeweils einer zugeordneten Vorderkante 20, ein annähernd kreissegmentförmiger Umriss der Stirnfläche 24 und drei Ecken 22 wie insbesondere anhand Figur 6 erkennbar ist.

Im dargestellten Fall, wirkt als primäres Werkzeug die Laserstrahlung 8. Dabei ist die Längsachse 15 der Skalpellendung 12 normal zur Oberfläche des Gewebes 13 ausgerichtet, so dass die Stirnfläche 24 parallel zur Oberfläche des Gewebes 13 liegt. Eine derartige Positionierung der Skalpellendung 12 ist jedoch lediglich beispielhaft. Die Stirnfläche 24 wird ferner in einem Abstand d über die Oberfläche des Gewebes 13 geführt. Die Abmessungen eines durch die Laserstrahlung 8 gebildeten Brennfleckes 29 sind zum einen von den Abmessungen der Stirnfläche 24 und zum anderen vom Abstand d der Stirnfläche 24 zur Oberfläche des Gewebes 13 abhängig. Grund dafür ist die bekannte Divergenz der Laserstrahlung 8, welche mit steigenden Abstand d den Brennfleck 29 vergrößert. Der Strahlaustritt erfolgt, wie dargestellt über die gesamte Stirnfläche 24. Das mögliche mechanische Einwirken der Skalpellendung 12 auf das zu behandelndes Gewebe 13, kommt in diesem Fall nicht zur Anwendung. Wie in Figur 5 schematisch dargestellt ist, ergibt sich eine sogenannte Koagulationszone 30, welche sowohl den Schnittgrund 31 als auch die Schnittflanken 32 umfasst. Oberhalb der Koagulationszone 30 befindet sich eine Karbonisationszone 33, die durch Wahl von Einstellparametern der Bearbeitungslichtquelle 6 auch nicht auftreten kann. Das dort befindliche Gewebe 13 kann durch die Laserstrahlung 8 entfernt werden.

Es sei angemerkt, dass bei dem in Figur 5 und 6 dargestellten Ausführungsbeispiel alle drei Ecken 22 auf der Außenseite 16 des Lichtleiters 3 liegen. Zum mechanischen Scheiden in eine Schneiderichtung S wird vor allem die Ecke 22 verwendet, in der sich die beiden Abschrägungsflächen 25 schneiden, da die Materialstärke D der Skalpellendung 12 in die Schneiderichtung S am größten ist. Es sei angemerkt, dass sich in der Ecke vier Kanten schneiden, nämlich die beiden Seitenkanten 26 und die beiden Vorderkanten 27. Ferner sei angemerkt, dass der Durchmesser und damit die Materialstärke D typischerweise 1,2 mm beträgt. Schließlich sei angemerkt, dass die Skalpellendung 12 typischerweise etwa 5 mm lang ist.

Bei einem in Figur 6 gestrichelt eingezeichneten, weiteren Abtrag der Abschrägungsflächen 25 kann sich eine in Längsrichtung des Lichtleiters 3 verlaufende Schnittfase 23 ergeben. In der Regel werden die Abschrägungsflächen 25 aber nur so stark abgetragen, dass sich die Ecke 22, von der die Schnittfase 23 ausgeht, in radiale Richtung näher an der Außenseite 16 des Lichtleiters 3 als an der Mitte M des Querschnitts 18 befindet.

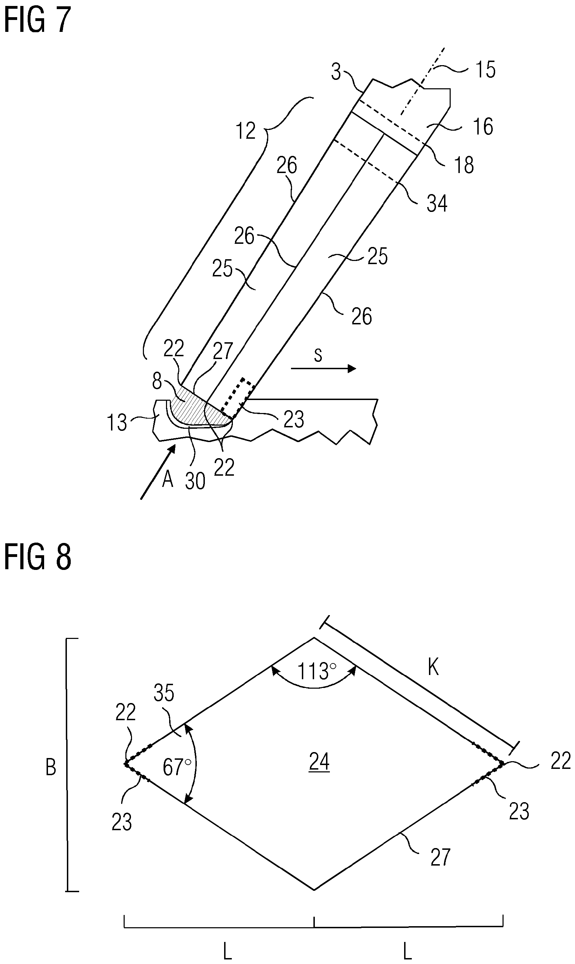

Wie weiters, insbesondere in den Figur 7 bis 10 verdeutlicht ist, ist vorteilhaft die, am distalen Ende 11 des Lichtleiters 3 normal zu dessen Längserstreckung liegende Stirnfläche 24 der Skalpellendung 12, als Polygon ausgeführt. Dabei wird durch zumindest eine Ecke 22 der Stirnfläche 24 eine am distalen Ende 11 des Lichtleiters 3 liegende, keilförmige Schnittfase 23 gebildet. In diesem Zusammenhang ist dabei unter keilförmig jede Art einer sich zur Schnittfase 23 hin verjüngenden Form zu verstehen.

Je nach gewählter Größe der Stirnfläche 24 und gewählter Polygonform ändert sich auch die Größe des Brennfleckes 29. Auf diese Weise kann auch eine Auswahl in Bezug auf die Fokussierung auf den zu behandelnden Bereich getroffen werden. Zum anderen ergibt sich durch eine keilförmige Schnittfase 23 eine vorteilhafte Geometrie, in Hinsicht auf die mechanische Schneidwirkung der Skalpellendung 12. Je nach zu behandelnden Gewebe 13 oder medizinischen Anwendungsgebiet, besteht mit dem erfindungsgemäßen optischen Skalpell 1 somit auch die Möglichkeit, die mechanische Schneidwirkung der Skalpellendung 12 zu nutzen. Dies erlaubt somit eine besonders vorteilhafte Nutzung des erfindungsgenmäßen optischen Skalpells 1 insbesondere für chirurgische Anwendungen, bei welchen zum einen eine mechanische Trennung von Gewebe 13 bevorzugt wird und zum anderen eine unmittelbare Blutgerinnung (Koagulation) erwünscht ist.

In diesem Zusammenhang ist Figur 7 eine Situation dargestellt, in welcher die zuvor erwähnte mechanische Schneidwirkung genutzt wird, um das Gewebe 13 entsprechend zu trennen. Eine bevorzugte Schneiderichtung ist dabei mit S gekennzeichnet. Die Skalpellendung 12 ist also so ausgeführt, dass auch eine mechanische Schnitteigenschaft, ähnlich dem eines klassischen Skalpells, erreicht wird. Weiterhin tritt die Laserstrahlung 8 überwiegend über die die Stirnfläche 24 aus und koaguliert unmittelbar hinter dem Schnitt durch die Schnittfase 23 die Blutung der Wunde.

Somit wird die Laserstrahlung 8 und die Erwärmung der Skalpellendung 12 mithilfe der Laserstrahlung 8 nicht nur zur Kauterisation oder Koagulation (Gerinnen) des beim Schneiden austretenden Blutes genutzt, so dass lediglich die Schnittkanten im Gewebe 13 koaguliert werden. Vielmehr wird die Schnittwunde auch dann vollständig koaguliert, wenn es zu einem aufklaffenden Schnittspalt kommt und sich der Schnittgrund 31 verbreitert. Die dadurch entstehende Oberfläche wird bei der Verwendung der hier beschriebenen Vorrichtung ausreichend koaguliert.

Ferner sei angemerkt, dass das Markierlicht 9 ebenso wie die Laserstrahlung 8 überwiegend über die Stirnfläche 24 austreten kann und dadurch den Behandlungsort zuverlässig markiert.

Weiter kann die Skalpellendung 12, so wie dies bereits im Zusammenhang mit Figur 5 beschrieben wurde, ebenso noch aus einem Abstand d von einem Gewebe 13 berührungsfrei als Bestrahlungsapplikator (zum Beispiel zur Punkt- und Flächenkoagulation oder zur Erwärmung eines definierten Körperareales) eingesetzt werden.

Der so ausgeführte Lichtleiter 3 mit der Skalpellendung 12 kann ferner kostengünstig hergestellt werden und hat einen einfachen Aufbau, durch welchen Energieverluste durch Kopplung mit anderen Materialien vermieden werden.

Wie bereits im Zusammenhang mit Figur 2 erwähnt, ist der derzeitige industrielle Standard, dass ein Lichtleiter 3 den kreisförmigen Querschnitt 18 aufweist. Bei der in Figur 7 dargestellten Variante, weisen lediglich die Skalpellendung 12 oder das distale Ende 11 des Lichtleiters 3, einen polygonen Querschnitt 34 auf. In einem beliebigen Abstand vom distalen Ende 11 des Lichtleiters 3 und dessen Stirnfläche 24 geht der polygone Querschnitt 34 der Skalpellendung 12 in den, beispielsweise kreisförmigen, Querschnitt 18 des Lichtleiters 3 über. Es ist auch möglich, dass der zumindest eine Lichtleiter 3 über seine gesamte Länge einen gleichbleibenden polygonen Querschnitt 34 aufweist. Zumindest eine Ecke 22 des polygonen Querschnittes 34, bildet dabei eine, zumindest annähernd in Richtung der Längserstreckung des Lichtleiters 3 liegende, keilförmige Schnittfase 23. Wiederum sei angemerkt, dass unter keilförmig jede Art einer sich zur Schnittfase 23 hin verjüngenden Form zu verstehen ist.

Dadurch kann eine bereits für den Lichtleiter 3 vorgewählte Form für die Skalpellendung 12 genutzt werden. Ein zuvor erwähntes, zusätzliches Bearbeiten jenes Bereichs, durch welchen die Skalpellendung 12 ausgebildet ist, ist nicht notwendig. Eine derartige Ausführung erlaubt weiter ein Kürzen des Lichtleiters 3, insbesondere an dessen zweitem Ende, ohne dass die zuvor beschriebene Geometrie verändert wird. Dies kann beispielsweise auch während eines chirurgischen Eingriffes, bei kurzer Unterbrechung der Prozedur, erfolgen. Auch das gekürzte zweite Ende des Lichtleiters 3 kann als Skalpellendung 12, mit den zuvor erwähnten Vorteilen weiter genutzt werden.

Wie ebenfalls in Figur 7 erkennbar ist, kann vorgesehen sein, dass die Stirnfläche 24 der Skalpellendung 12 derart ausgestaltet ist, dass zumindest zwei keilförmige Schnittfasen 23 mit zumindest annähernd gleichem oder unterschiedlichem Keilwinkel 35 gebildet sind. In Figur 8 ist eine derartige rautenförmige Stirnfläche 24 dargestellt. Typische Abmessungen sind für die Breite B = 0,8 mm, für die Länge L = 0,6 mm und für die Kantenlänge K = 0,7 mm. Bei zwei gleichartigen Keilwinkel 35 ergibt sich der Vorteil der richtungsunabhängigen Nutzung.

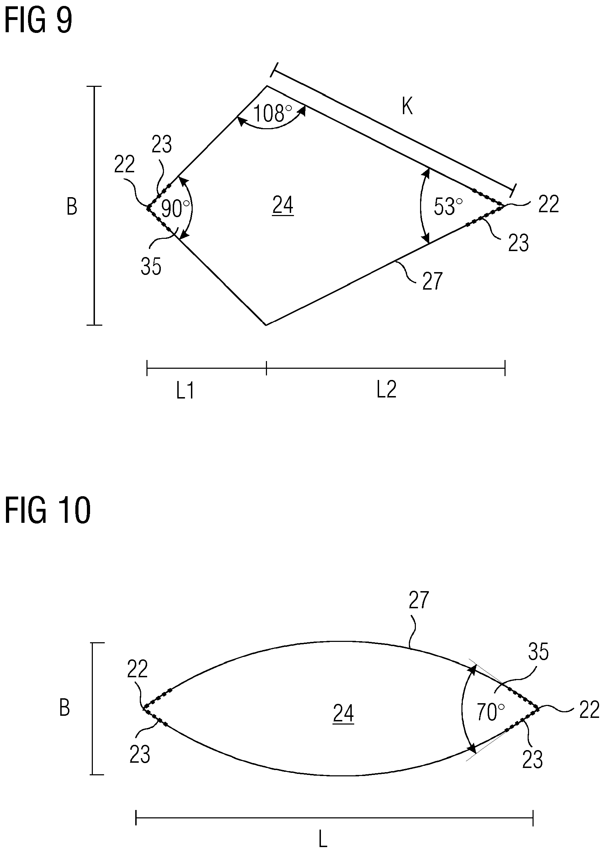

Weist die Skalpellendung 12 dagegen unterschiedliche Keilwinkel 35 auf, kann an der Skalpellendung 12 der jeweilige Keilwinkel 35 beispielsweise für unterschiedliche Verfahren zur Anwendung kommen. In diesem Zusammenhang sei beispielsweise auf die Ausführungsvarianten, welche in den Figuren 9 und 10 dargestellt sind, hingewiesen. Dabei handelt es sich lediglich um beispielhafte Ausführungsvarianten der Stirnfläche 24. Beispielsweise könnte ein polygoner Querschnitt 34 auch derart ausgeführt sein, dass sich eine doppelte Schnittfase 23 bildet, bei welchen zwei gleiche oder auch unterschiedliche Keilwinkel 35 mit dem Gewebe 13 wechselwirken. Figur 9 zeigt ein Ausführungsbeispiel, dessen Stirnfläche 24 einen deltoidförmigen Querschnitt aufweist. In Figur 9 sind typische Abmessungen für die Breite B = 0,8 mm, für die Längen L1= 0,4 mm und Länge L2 = 0,8 mm sowie für die Kantenlänge K = 0,9 mm. Figur 10 zeigt schließlich ein Ausführungsbeispiel, dessen Stirnfläche 24 einen mandelförmigen Querschnitt aufweist. Typische Abmessungen sind für die Breite B 0,4 mm und für die Länge L = 1,2 mm.

Für den zuvor erwähnten Keilwinkel 35 einer keilförmigen Schnittfase 23 kommt vorteilhaft ein Bereich zwischen 5° und 120°, insbesondere zwischen 25° und 50° in Betracht. In nachvollziehbarer Weise, wird durch die entsprechende Auswahl des Keilwinkels 35, die zuvor erwähnte mechanische Schneidwirkung unterstützt.

Es sei angemerkt, dass bei den in den Figuren 3 bis 10 dargestellten Ausführungsbeispielen mit Stirnflächen 24, die Stirnflächen 24 jeweils im rechten Winkel zur Längsachse 15 ausgerichtet sind. Bei weiteren abgewandelten Ausführungsbeispielen können die Stirnflächen 24 so ausgerichtet sein, dass die Laserstrahlung 8 und das Markierlicht 9 beim Durchgang durch die Stirnfläche 24 zur Schnittfase 23 hin gebrochen werden, um zu erreichen, dass die Koagulationszone 30 näher an der Skalpellendung 12 zum Liegen kommt. Denkbar sind insbesondere Neigungswinkel der Stirnfläche 24 zur Längsachse 15 im Bereich von 45° bis 90°.

Im Regelfall wird die Stirnfläche 24 aber einen Winkel zwischen 80° und 90° zur Längsachse 15 einnehmen.

Durch die vorliegende Erfindung wird ein chirurgisches Instrument auf Basis eines Lichtleiters 3 ausgebildet. Neben der Nutzung als Skalpell, kann dieses auch wahlweise als Kauter genutzt werden. Der einfache Aufbau erlaubt eine kostengünstige Herstellung. Weiters reduziert der erfindungsgemäße Aufbau Einkoppelverluste und Reinigungs- und Sterilisationsaufwand.

## Patentansprüche

1. Optisches Skalpell (1) mit zumindest einem Lichtleiter (3), der ein proximales Ende (4) und ein dem proximalen Ende (4) entgegengesetztes distales Ende (11) aufweist und dessen proximales Ende (4) an zumindest eine Bearbeitungslichtquelle (6) anschließbar ist,
**dadurch gekennzeichnet, dass**
das distale Ende (11) des Lichtleiters (3) als Skalpellendung (12) ausgebildet ist, wobei am distalen Ende (11) des Lichtleiters (3) eine quer zu einer Längsachse (15) des Lichtleiters ausgerichtete Stirnfläche (24) ausgebildet ist und wobei das distale Ende (11) zumindest eine vom distalen Ende (11) des Lichtleiters (3) ausgehende, seitliche Schneidekante (21, 26) aufweist.

2. Optisches Skalpell (1) nach Anspruch 1,
bei dem die Skalpellendung (12) eine am distalen Ende (11) des Lichtleiters (3) angeordnete Ecke (22) aufweist, in der sich zumindest drei Kanten (20, 21, 26, 27, 28) scheiden, von denen zumindest eine als Schneidekante (21, 26) ausgebildet ist, die sich von der Ecke (22) aus wenigstens abschnittsweise in Richtung des proximalen Endes (4) des Lichtleiters (3) erstreckt.

3. Optisches Skalpell (1) nach Anspruch 2,
bei dem im Querschnitt (18, 34) des Lichtleiters (3) die Ecke (22) der Skalpellendung (12) weiter von der Mitte des Lichtleiters (3) entfernt ist als von der Außenseite (16) des Lichtleiters (3).

4. Optisches Skalpell (1) nach Anspruch 3,
bei dem sich die Ecke (22) der Skalpellendung (12) im Querschnitt (18, 34) auf der Außenseite (16) des Lichtleiters (3) befindet.

5. Optisches Skalpell (1) nach einem der Ansprüche 1 bis 4,
bei dem die Skalpellendung (12) eine vom distalen Ende (11) des Lichtleiters (3) ausgehende Schnittfase (23) aufweist, die sich im Querschnitt (18, 34) des Lichtleiters (3) zu der Schneidekante (21, 26) hin verjüngt.

6. Optisches Skalpell (1) nach einem der Ansprüche 1 bis 5,
bei dem die Skalpellendung (12) durch eine sich in Längsrichtung erstreckende keilförmige Verjüngung des Lichtleiters (3) ausgebildet ist und durch zumindest eine dabei gebildete Ecke (22) die Spitze der Skalpellendung (12) bildet, von der aus eine am distalen Ende (11) des Lichtleiters (3) liegende Schnittfase (23) ausgeht.

7. Optisches Skalpell (1) nach einem der Ansprüche 1 bis 6,
bei dem eine am distalen Ende (11) des Lichtleiters (3) normal zu dessen Längsachse ausgerichtete Stirnfläche (24) der Skalpellendung (12), als Polygon ausgeführt ist und zumindest eine Ecke (22) der Stirnfläche (24) die Ecke der Skalpellendung (12) bildet, von der aus die am distalen Ende (11) des Lichtleiters (3) liegende Schnittfase (23) ausgeht.

8. Optisches Skalpell (1) nach Anspruch 7,
bei dem der zumindest eine Lichtleiter (3) über seine gesamte Länge einen gleichbleibenden polygonen Querschnitt (34) aufweist und zumindest eine Ecke (22) des polygonen Querschnittes (34) die Ecke der Skalpellendung (12) bildet, von der aus die sich entlang der Längsachse (15) des Lichtleiters (3) erstreckende Schnittfase (23) ausgeht.

9. Optisches Skalpell (1) nach Anspruch 7 oder 8,
bei dem von der Stirnfläche (24) der Skalpellendung (12) zumindest zwei Schnittfasen (23) ausgehen.

10. Optisches Skalpell (1) nach einem der Ansprüche 5 bis 9,
bei dem die Schnittfase (23) keilförmig ausgebildet ist und der Keilwinkel (35) der keilförmigen Schnittfase (23) zwischen 5° und 120°, insbesondere zwischen 25° und 50° gewählt ist.

11. Schneidevorrichtung (2) für die Chirurgie mit zumindest einer Bearbeitungslichtquelle (6) und wenigstens einem daran angeschlossenen optischen Skalpell (1), **dadurch gekennzeichnet, dass**
das optische Skalpell (1) nach einem der Ansprüche 1 bis 10 ausgebildet ist.

12. Schneidevorrichtung (2) für die Chirurgie nach Anspruch 11,
bei der die zumindest eine Bearbeitungslichtquelle (6) durch einen Bearbeitungslaser (6) gebildet ist.

13. Schneidevorrichtung (2) nach Anspruch 11 oder 12,
bei der zumindest eine weitere Markierlichtquelle (7), zur Markierung eines Behandlungsbereiches auf der Oberfläche eines zu behandelnden Gewebes (13) vorgesehen ist.

14. Schneidevorrichtung (2) nach Anspruch 13,
bei der das von der Markierlichtquelle (7) ausgehende Markierlicht (9) mit Hilfe einer Koppelvorrichtung (10) zusammen mit der Laserstrahlung (8) dem Lichtleiter (3) zugeführt ist.

## Claims

1. An optical scalpel (1) with at least one light guide (3) having a proximal end (4) and a distal end (11) opposite the proximal end (4), and whose proximal end (4) can be connected to at least one processing light source (6), **characterized in that**
the distal end (11) of the light guide (3) is configured as a scalpel end (12), wherein an end face (24) oriented transversely to a longitudinal axis (15) of the light guide is formed at the distal end (11) of the light guide (3), and wherein the distal end (11) has at least one lateral cutting edge (21, 26) emanating from the distal end (11) of the light guide (3).

2. The optical scalpel (1) according to claim 1,
wherein the scalpel end (12) has a corner (22), which is disposed at the distal end (11) of the light guide (3) and at which at least three edges (20, 21, 26, 27, 28) intersect, at least one of which is configured as a cutting edge (21, 26) that extends at least in some sections from the corner (22) in the direction of the proximal end (4) of the light guide (3).

3. The optical scalpel (1) according to claim 2,
wherein, in the cross section (18, 34) of the light guide (3), the corner (22) of the scalpel end (12) is farther distant from the center of the light guide (3) than from the outer side (16) of the light guide (3).

4. The optical scalpel (1) according to claim 3,
wherein, in the cross section (18, 34), the corner (22) of the scalpel end (12) is located on the outer side (16) of the light guide (3).

5. The optical scalpel (1) according to any one of the claims 1 to 4,
wherein the scalpel end (12) has a cutting bevel (23), which emanates from the distal end (11) of the light guide (3) and, in the cross section (18, 34) of the light guide (3), tapers towards the cutting edge (21, 26).

6. The optical scalpel (1) according to any one of the claims 1 to 5,
wherein the scalpel end (12) is formed by a longitudinally extending, wedge-shaped, tapered portion of the light guide (3) and, by means of at least one corner (22) formed thereby, forms the tip of the scalpel end (12) from which a cutting bevel (23), which is located at the distal end (11) of the light guide (3), emanates.

7. The optical scalpel (1) according to any one of the claims 1 to 6,
wherein an end face (24) of the scalpel end (12) which, at the distal end (11) of the light guide (3), is oriented normally to the longitudinal axis thereof, is configured as a polygon, and at least one corner (22) of the end face (24) forms the corner of the scalpel end (12) from which the cutting bevel (23), which is located at the distal end (11) of the light guide (3), emanates.

8. The optical scalpel (1) according to claim 7,
wherein the at least one light guide (3) has an unvarying polygonal cross section (34) over its entire length, and at least one corner (22) of the polygonal cross section (34) forms the corner of the scalpel end (12) from which the cutting bevel (23), which extends along the longitudinal axis (15) of the light guide (3), emanates.

9. The optical scalpel (1) according to claim 7 or 8,
wherein at least two cutting bevels (23) emanate from the end face (24) of the scalpel end (12).

10. The optical scalpel (1) according to any one of the claims 5 to 9,
wherein the cutting bevel (23) has a wedge-shaped configuration, and the wedge angle (35) of the wedge-shaped cutting bevel (23) is selected to be between 5° and 120°, in particular between 25° and 50°.

11. A surgical cutting device (2) with at least one processing light source (6) and at least one optical scalpel (1) connected thereto, **characterized in that** the optical scalpel (1) is configured in accordance with any one of the claims 1 to 10.

12. The surgical cutting device (2) according to claim 11,
wherein the at least processing light source (6) is formed by a processing laser (6).

13. The surgical cutting device (2) according to claim 11 or 12,
wherein at least one further marking light source (7) for marking a treatment site on the surface of a tissue (13) to be treated is provided.

14. The surgical cutting device (2) according to claim 13,
wherein the marking light (9) emanating from the marking light source (7) is fed to the light guide (3), together with the laser radiation (8), using a coupling device (10).

## Revendications

1. Scalpel optique (1) avec au moins un conducteur optique (3) qui présente une extrémité proximale (4) et une extrémité distale (11) opposée à l'extrémité proximale (4), et dont l'extrémité proximale (4) peut être raccordée à au moins une source lumineuse d'usinage (6),
**caractérisé en ce que**
l'extrémité distale (11) du conducteur optique (3) est réalisée en tant que terminaison de scalpel (12), dans lequel une face frontale (24) orientée transversalement à un axe longitudinal (15) du conducteur optique est réalisée à l'extrémité distale (11) du conducteur optique (3), et dans lequel l'extrémité distale (11) présente au moins une arête de coupe latérale (21, 26) partant de l'extrémité distale (11) du conducteur optique (3).

2. Scalpel optique (1) selon la revendication 1,
dans lequel la terminaison de scalpel (12) présente un coin (22) disposé à l'extrémité distale (11) du conducteur optique (3), dans lequel au moins trois arêtes (20, 21, 26, 27, 28) se coupent, parmi lesquelles au moins une est réalisée en tant qu'arête de coupe (21, 26) qui s'étend du coin (22) au moins par sections en direction de l'extrémité proximale (4) du conducteur optique (3).

3. Scalpel optique (1) selon la revendication 2,
dans lequel le coin (22) de la terminaison de scalpel (12) est plus éloigné du milieu du conducteur optique (3) que du côté externe (16) du conducteur optique (3) dans la section transversale (18, 34) du conducteur optique (3).

4. Scalpel optique (1) selon la revendication 3,
dans lequel le coin (22) de la terminaison de scalpel (12) se trouve dans la section transversale (18, 34) sur le côté externe (16) du conducteur optique (3).

5. Scalpel optique (1) selon une des revendications 1 à 4,
dans lequel la terminaison de scalpel (12) présente un chanfrein de coupe (23) partant de l'extrémité distale (11) du conducteur optique (3), qui se rétrécit dans la section transversale (18, 34) du conducteur optique (3) vers l'arête de coupe (21, 26).

6. Scalpel optique (1) selon une des revendications 1 à 5,
dans lequel la terminaison de scalpel (12) est réalisée par un rétrécissement en forme de cale du conducteur optique (3) s'étendant dans la direction longitudinale et forme par au moins un coin formé ce faisant (22) la pointe de la terminaison de scalpel (12), de laquelle part un chanfrein de coupe (23) situé à l'extrémité distale (11) du conducteur optique (3).

7. Scalpel optique (1) selon une des revendications 1 à 6,
dans lequel une face frontale (24) de la terminaison de scalpel (12) orientée à l'extrémité distale (11) du conducteur optique (3) perpendiculairement à son axe longitudinal est réalisée en tant que polygone et au moins un coin (22) de la face frontale (24) forme le coin de la terminaison de scalpel (12), duquel part le chanfrein de coupe (23) situé à l'extrémité distale (11) du conducteur optique (3).

8. Scalpel optique (1) selon la revendication 7,
dans lequel au moins un conducteur optique (3) présente sur sa longueur entière une section transversale polygonale constante (34) et au moins un coin (22) de la section transversale polygonale (34) forme le coin de la terminaison de scalpel (12), duquel part le chanfrein de coupe (23) s'étendant le long de l'axe longitudinal (15) du conducteur optique (3).

9. Scalpel optique (1) selon la revendication 7 ou 8,
dans lequel au moins deux chanfreins de coupe (23) partent de la face frontale (24) de la terminaison de scalpel (12).

10. Scalpel optique (1) selon une des revendications 5 à 9,
dans lequel le chanfrein de coupe (23) est réalisé en forme de cale et
l'angle de cale (35) du chanfrein de coupe en forme de cale (23) est choisi entre 5° et 120°, notamment entre 25° et 50°.

11. Dispositif de coupe (2) pour la chirurgie avec au moins une source lumineuse d'usinage (6) et au moins un scalpel optique (1) raccordé à celle-ci, **caractérisé en ce que**
le scalpel optique (1) est réalisé selon une des revendications 1 à 10.

12. Dispositif de coupe (2) pour la chirurgie selon la revendication 11, dans lequel l'au moins une source lumineuse d'usinage (6) est formée par un laser d'usinage (6).

13. Dispositif de coupe (2) selon la revendication 11 ou 12,
dans lequel au moins une autre source lumineuse de marquage (7) est prévue pour le marquage d'une région de traitement sur la surface d'un tissu à traiter (13).

14. Dispositif de coupe (2) selon la revendication 13,
dans lequel la lumière de marquage (9) partant de la source lumineuse de marquage (7) est acheminée au conducteur optique (3) à l'aide d'un dispositif de couplage (10) en combinaison avec le rayonnement laser (8).
